# EUROPÄISCHE PATENTANMELDUNG

(11) **EP 3 865 592 A2**
(43) Veröffentlichungstag der Anmeldung: **18.08.2021**
(21) Anmeldenummer: 21157063.5
(22) Anmeldetag: 15.02.2021
(51) Int. Cl.: C12Q 1/6888, C12Q 1/689, C12Q 1/6841

(54) **OLIGONUKLEOTIDSONDE ZUM SPEZIFISCHEN NACHWEIS VON MIKROORGANISMEN, KORRESPONDIERENDES VERFAHREN UND VERWENDUNG**

(30) Priorität: 14.02.2020 DE 102020103958; 12.02.2021 US 202117174799
(71) Anmelder: Testo bioAnalytics GmbH, 79822 Titisee-Neustadt (DE)
(72) Erfinder: Quehl, Paul, 37085 Göttingen (DE); Vasileuskaya-Schulz, Zinaida, 79111 Freiburg (DE); Boos, Stefanie, 78052 Villingen-Schwenningen (DE)
(74) Vertreter: Mertzlufft-Paufler, Cornelius

(57) **Zusammenfassung**

Die Erfindung betrifft eine Oligonukleotidsonden-Kombination (1) zum spezifischen Nachweis von Mikroorganismen, mit einer Primärsonde (5) und einer Sekundärsonde (6), wobei die Primärsonde (5) wenigstens einen Funktionsabschnitt (2) und die Sekundärsonde (6) wenigstens eine ein detektierbares Signal erzeugende Markierung (3) aufweist, wobei der Funktionsabschnitt (2) an eine Zielsequenz eines nachzuweisenden Mikroorganismus spezifisch anbindet, wobei die Primärsonde (5) und die Sekundärsonde (6) miteinander verbunden sind aber getrennte Ketten (8) darstellen.

## Beschreibung

Die Erfindung betrifft eine Oligonukleotidsonden-Kombination zum spezifischen Nachweis von Mikroorganismen, insbesondere zur Detektion von wenigstens einer Zielsequenz, mit wenigstens einem Funktionsabschnitt und wenigstens einer detektierbaren Markierung, insbesondere einer Farbmarkierung, wobei der wenigstens eine Funktionsabschnitt an die Zielsequenz von Mikroorganismen der nachzuweisenden Gruppe spezifisch anbindet und durch eine Interaktion mit der wenigstens einen Markierung ein detektierbares Signal auslöst.

Zum spezifischen Nachweis von Nukleinsäuren, d.h. DNA und/oder RNA Molekülen in einzelnen Zellen sind beispielsweise Verfahren wie In-Situ-Hybridisierung (ISH) bekannt. Dabei werden kurze synthetische Nukleinsäuresonden eingesetzt, welche über komplementäre Basenpaarungen an die nachzuweisende Zielsequenz binden. Eine Variante der ISH-Technologie, bei welcher die Nukleinsäuresonden fluoreszenzmarkiert sind, ist die Fluoreszenz-In-Situ-Hybridisierung (FISH).

Bei den bekannten FISH-Verfahren wird die Zellhülle der in der Probe enthaltenen Mikroorganismen für Nukleinsäuresonden permeabilisiert. Die Nukleinsäuresonden, die aus einem Oligonukleotid und einem daran gebundenen Marker bestehen, können dann die Zellhülle durchdringen und sich an die Zielsequenz im Zellinneren binden. Durch eine zusätzliche Fixierung bleibt die Zellstruktur erhalten.

Wegen der sequenzabhängigen, hohen Variabilität der chemischphysikalischen Eigenschaften von Oligonukleotiden muss für jedes Oligonukleotid gerade bei größeren Produktionsmaßstäben ein kosten- und zeitintensiver Entwicklungsprozess für dessen Herstellung durchlaufen werden. Dabei steigt der Aufwand linear mit der Anzahl der im Assay notwendigen Oligonukleotiden. Für jede nachzuweisende Zielsequenz wird dabei ein jeweiliges Fluoreszenzfarbstoff-modifiziertes Oligonukleotid verwendet. Dadurch können je nach nachzuweisendem Mikroorganismus unterschiedlich viele, teilweise deutlich mehr als 25, Oligonukleotiden notwendig sein. Auf diese Weise wird das experimentelle Screening nach neuen oder besseren Oligonukleotid-Varianten mit jeweils mindestens einer Fluoreszenzfarbstoff-Modifikation sehr teuer, da jede Variante mit einem Fluoreszenzfarbstoff modifiziert werden muss. Von Vorteil ist dabei, dass eine einfache und kostengünstige Analytik erreichbar ist, die eine gleichzeitige Bestimmung mehrerer Zielsequenzen in einem Assay gewährleisten kann.

Vor diesem Hintergrund ist es Aufgabe der vorliegenden Erfindung, eine Oligonukleotidsonden-Kombination zum spezifischen Nachweis von Zielsequenzen, d.h. DNA- oder RNA-Sequenzen in Mikroorganismen bereitzustellen, die eine spezifische Detektion von mehreren verschiedenen Zielsequenzen in einem FISH-Verfahren ermöglicht, das kostengünstig und außerhalb von Laborumgebungen durchführbar ist.

Erfindungsgemäß wird diese Aufgabe durch die Merkmale von Anspruch 1 gelöst.

Insbesondere wird somit erfindungsgemäß zur Lösung der genannten Aufgabe bei einer Oligonukleotidsonden-Kombination der eingangs beschriebenen Art vorgeschlagen, dass wenigstens ein Funktionsabschnitt und wenigstens eine detektierbare Markierung, insbesondere eine Farbmarkierung, verwendet werden, wobei der wenigstens eine Funktionsabschnitt an die Zielsequenz von Mikroorganismen der nachzuweisenden Gruppe spezifisch anbindet und durch eine Interaktion mit der wenigstens einen Markierung ein detektierbares Signal auslöst. Die erfindungsgemäße Oligonukleotidsonden-Kombination weist wenigstens eine Primärsonde und wenigstens eine Sekundärsonde auf, die, vorzugsweise über komplementäre Basenpaare, miteinander verbunden sind, aber getrennte Ketten darstellen, wobei an der wenigstens einen Primärsonde der wenigstens eine Funktionsabschnitt und an der wenigstens einen Sekundärsonde die wenigstens eine Markierung ausgebildet sind. Somit ist einfach erreichbar, den Prozess der Markierung, insbesondere der Farbmarkierung, zu vereinheitlichen, da immer die gleiche Sequenz als Sekundärsonde verwendbar ist. Vorteilhaft dabei ist, dass somit die Detektierbarkeit von Mikroorganismen erheblich verbessert werden kann, da mit der Kombination mehrerer Oligonukleotidsonden eine höhere Fluoreszenzintensität pro Bakterium erreichbar ist, indem mehrere Zielsequenzen gleichzeitig nachgewiesen werden können.

Nachfolgend werden vorteilhafte Ausgestaltungen der Erfindung beschrieben, die allein oder in Kombination mit den Merkmalen anderer Ausgestaltungen optional zusammen mit den Merkmalen nach Anspruch 1 kombiniert werden können.

Eine vorteilhafte Ausführungsform gemäß der Erfindung sieht vor, dass die wenigstens eine Primärsonde als lineare Sonde ausgebildet ist. Beispielsweise sind das monoProbes, dopeProbes, tetraProbes und multiProbes. Alternativ oder zusätzlich kann die Primärsonde als eine Sonde mit Sekundärstruktur, vorzugsweise eine Haarnadel-Sonde, ausgebildet sein. Beispielsweise sind das Molecular Beacons und Scorpions Sonden. Dadurch sind eine höhere Fluoreszenzintensität sowie ein besseres Signal-Rausch-Verhältnis erreichbar, was insbesondere für eine automatisierte Anwendung vorteilhaft ist.

Eine vorteilhafte Ausführungsform gemäß der Erfindung sieht vor, dass die wenigstens eine Primärsonde einen Stammabschnitt aufweist, der zur Bildung der Haarnadel-Struktur ausgebildet ist. Die Erfindung macht sich hierbei zunutze, dass die Stammbildenden Nukleotide in Abwesenheit von Zielsequenzen eine "Haarnadel"-Struktur bilden können, wodurch ein Signal der Markierung, wie die Fluoreszenz eines Farbstoffs, unterdrückt wird. Die Unterdrückung des Signals der Markierung kann beispielsweise in Anwesenheit eines Quencher-Moleküls erfolgen. Nach der Bindung des Funktionsabschnitts an die Zielsequenz löst sich diese "Haarnadel"-Struktur wieder auf, wobei das Signal der Markierung, insbesondere die Fluoreszenz des Farbstoffs, das nicht mehr unterdrückt wird, detektiert werden kann.

Eine vorteilhafte Ausführungsform gemäß der Erfindung sieht vor, dass die wenigstens eine Primärsonde wenigstens einen Köderabschnitt aufweist, der mit dem Stammabschnitt übereinstimmend oder separat, insbesondere beabstandet, von dem Stammabschnitt ausgebildet ist. Dadurch ist eine flexible Gestaltung in der Sequenzlänge des Köderabschnitts erreichbar, die an der Sequenz der Sekundärsonde angepasst bzw. optimiert werden kann.

Eine vorteilhafte Ausführungsform gemäß der Erfindung sieht vor, dass der Köderabschnitt an wenigstens einem Ende der wenigstens einen Primärsonde ausgebildet ist. Somit kann mindestens eine Bindungsstelle für die mit einer Markierung versehene Sekundärsonde ermöglicht werden. Alternativ kann der Köderabschnitt an beiden Enden der wenigstens einen Primärsonde ausgebildet sein. Dadurch sind eine Signalverstärkung und eine höhere Fluoreszenzintensität erreichbar, da eine Bindung zweier Sekundärsonden an eine Primärsonde ermöglicht werden kann. Vorteilshaft ist dabei, dass wenn zwei Köderabschnitte pro Primärsonde vorhanden sind, zwei Sekundärsonden mit jeweils zwei Farbmarkierungen, beispielsweise Fluoreszenzmarkierungen, binden können. Somit können mehrere Farbmarkierungen pro Zielsequenz genutzt werden. Zusätzlich ist durch die Ausbildung eines Köderabschnitts an einem oder an beiden Enden der Primärsonde und nicht beispielsweise in deren Mitte eine bessere Stabilität der Oligonukleotidsonde erreichbar.

Eine vorteilhafte Ausführungsform gemäß der Erfindung sieht vor, dass die wenigstens eine Primärsonde einen Quencher (Signallöscher, insbesondere Fluoreszenzlöscher) aufweist, der zur Deaktivierung der Markierung der Sekundärsonde ausgebildet ist. Vorteilhaft ist dabei, dass keine Fluoreszenzsignale von Oligonukleotidsonden emittiert werden, die nicht an eine Zielsequenz gebundenen sind. Dadurch ist ein verbessertes Signal-Rausch-Verhältnis erreichbar.

Eine vorteilhafte Ausführungsform gemäß der Erfindung sieht vor, dass die wenigstens eine Primärsonde einen Köderabschnitt aufweist, an den die Sekundärsonde anbindet. Es kann insbesondere vorgesehen sein, dass die wenigstens eine Primärsonde wenigstens ein modifiziertes Nukleotid aufweist. Die Modifikationen können die Zuckerreste der Nucleotide (beispielsweise Anhängen von Fluoreszenz-Gruppen, Schwermetall-Ionen oder Amino-Linkern), die Basen (beispielsweise Thio-Basenanaloga, Fluoreszenz-Gruppen, ungewöhnliche Basen) oder die Phosphat-Reste (beispielsweise nicht-hydrolyiserbare Derivate, photolabile Gruppen) betreffen. Vorzugsweise sind die modifizierten Nukleotide sogenannte "Locked nucleid acids" (LNA)-Nukleotide. Die LNA-Bausteine sollten dabei nicht selbst-komplementär sein. Durch den Einbau von einem oder mehreren modifizierten Nukleotiden, beispielsweise LNA-Nukleotiden, kann die Bindungsaffinität der Oligonukleotidsonde erhöht werden. Von Vorteil ist dabei, dass durch die erhöhte Bindungsaffinität die Länge der Oligonukleotidsonde verkürzt und somit eine kostengünstigere Oligonukleotidsonde bereitgestellt werden kann.

Eine vorteilhafte Ausführungsform gemäß der Erfindung sieht vor, dass die Sekundärsonde aus DNA-Nukleotiden oder aus einer Mischung von DNA-Nukleotiden und wenigstens einem modifizierten Nukleotid besteht. Vorzugsweise ist das wenigstens ein modifiziertes Nukleotid ein LNA-Nukleotid. Vorteilhaft ist dabei, dass die modifizierten Nukleotide, insbesondere LNA-Nukleotide, die komplementäre Basenpaarung zwischen der Sekundärsonde und dem Köderabschnitt der Primärsonde verstärken können, so dass stabilere Dimere entstehen können, die ein besseres Signal ermöglichen. Es ist weiterhin vorgesehen, dass das wenigstens ein modifiziertes Nukleotid der Sekundärsonde und das wenigstens ein modifiziertes Nukleotid der Primärsonde untereinander nicht komplementär ausgebildet sind.

Eine vorteilhafte Ausführungsform gemäß der Erfindung sieht vor, dass die Sekundärsonde eine Länge von weniger als 25 Nukleinbasen aufweist. Dadurch kann ein zu starkes Bindungsverhalten vermieden sowie die Bereitstellung einer kostengünstigeren Oligonukleotidsonde mit besseren Diffusionseigenschaften ermöglicht werden.

Eine vorteilhafte Ausführungsform gemäß der Erfindung sieht vor, dass die wenigstens eine Markierung einen Marker umfasst. Alternativ kann die Markierung eine Kombination aus wenigstens zwei Markern umfassen. Es kann vorgesehen sein, dass der Marker eine Fluoreszenz-basierte, Biotin- und/oder Radioaktivitätsmarkierung umfasst. Somit ist eine optische Detektion erreichbar. Alternativ oder zusätzlich kann der Marker einen Affinitätsmarker und/oder eine enzymatisch aktive Gruppe umfassen. Der Affinitätsmarker kann beispielsweise Affinitätsbindungspaare Biotin-Streptavidin oder Antigen-Antikörper umfassen.

Es kann auch vorgesehen sein, dass die Markierung einen Bestandteil aufweist, der aus Peroxidase, vorzugsweise Meerrettich-Peroxidase, und/oder Phosphatase, vorzugsweise alkalischer Phosphatase, ausgewählt ist.

Eine vorteilhafte Ausführungsform gemäß der Erfindung sieht vor, dass die wenigstens eine Zielsequenz eine oder mehrere verschiedene DNA- und/oder RNA-Sequenz/Sequenzen umfasst. Somit kann ein spezifischer Nachweis von Mikroorganismen auf DNA- und/oder RNA-Ebene ermöglicht werden.

Ein weiterer Gegenstand der Erfindung ist ein Verfahren zum spezifischen Nachweis von Mikroorganismen in einer Probe mittels In-Situ-Hybridisierung (ISH), insbesondere Fluoreszenz-In-Situ-Hybridisierung (FISH), umfassend die folgenden Schritte: a) Permeabilisierung und Fixierung der in der Probe enthaltenen Mikroorganismen; b) Inkubieren der fixierten Mikroorganismen mit wenigstens einer Oligonukleotidsonden-Kombination, insbesondere wie zuvor beschrieben und/oder nach einem der auf eine Oligonukleotidsonden-Kombination gerichteten Ansprüche, wobei die einzelne Oligonukleotidsonden-Kombination mit mehreren verschiedenen Nukleinsäuren der Mikroorganismen eine komplementäre Hybridisierung eingeht; c) vorzugsweise optisches Detektieren der in den einzelnen Mikroorganismen erzeugten Hybridisierungen. Die optische Detektion kann durch Fluoreszenz- oder durch beispielsweise Luciferasemessung, vorzugsweise Renilla-luceferin-2-monooxygenase, erfolgen.

Es kann vorgesehen sein, dass vor dem optischen Detektieren der erzeugten Hybridisierungen zusätzlich ein Waschschritt ausgeführt wird. Dadurch können die nicht hybridisierten Nukleinsäuresonden entfernt und somit ein verbessertes Signal-Rausch-Verhältnis ermöglicht werden.

Eine vorteilhafte Ausführungsform gemäß der Erfindung sieht weiterhin vor, dass das Detektieren einen Schritt des Quantifizierens mit hybridisierten Oligonukleotidsonden umfasst. Dadurch ist zusätzlich zu einer qualitativen Aussage auch eine absolute Quantifizierung der nachzuweisenden Mikroorganismen beispielsweise auf Basis einer Partikelmessung erreichbar.

Eine vorteilhafte Ausführungsform gemäß der Erfindung sieht weiterhin vor, dass jede Oligonukleotidsonden-Kombination nachweisbar nur an die komplementären Zielsequenzen bindet. Somit ist eine hoch spezifische Nachweisbarkeit der gewünschten Mikroorganismen erreichbar.

Eine bevorzugte Anwendung sieht eine Verwendung wenigstens einer Oligonukleotidsonden-Kombination, insbesondere wie zuvor beschrieben und/oder nach einem der auf eine Oligonukleotidsonden-Kombination gerichteten Ansprüche, zum spezifischen Nachweis von mehreren verschiedenen Nukleinsäuresequenzen, insbesondere wobei der Nachweis in einer natürlichen Probe ausgewählt aus klinischen Proben, Lebensmittelproben, Umweltproben, Umgebungsproben und veterinärdiagnostischen Proben oder in einer Laborkultur erfolgt. Von Vorteil ist dabei, dass ein spezifischer Nachweis von Mikroorganismen in unterschiedlichen Applikationsfeldern ermöglicht werden kann.

Eine bevorzugte Anwendung sieht eine Verwendung eines Probenträgers, insbesondere wie zuvor beschrieben und/oder nach einem der auf ein Verfahren gerichteten Ansprüche und/oder nach einem auf eine Verwendung gerichteten Anspruch.

Der erfindungsgemäße Probenträger kann als ein scheibenförmiger Probenträger ausgebildet sein. Beispielsweise kann der Probenträger als ein flacher Probenträger ausgebildet sein. Von Vorteil ist dabei, dass durch die Scheibenform des Probenträgers die Zentrifugalkraft für die Fluidförderung ausgenutzt werden kann. Die Fluidförderung ist auch über Druck oder auf eine andere Weise erreichbar. Der Probenträger kann alternativ eine dreidimensionale Erstreckung, beispielsweise in Form eines Zylinders oder küvettenartig, aufweisen.

Beispielsweise kann die Scheibenförmigkeit rotationssymmetrisch ausgebildet sein. Dies kann für ein Zentrifugieren von Vorteil sein. Es sind auch alternativ rechteckförmige Probenträger wie bei einer Chipkarte oder segmentförmige Probenträger wie bei einem Pizzastück bildbar.

Beispielsweise können bei dem erfindungsgemäßen Verfahren die Permeabilisierung und Fixierung und/oder die Hybridisierung und/oder das Detektieren der Markierung im Probenträger erfolgen.

Die Erfindung wird nun anhand von Ausführungsbeispielen näher beschrieben, ist aber nicht auf diese Ausführungsbeispiele beschränkt. Weitere Ausführungsbeispiele ergeben sich durch Kombination der Merkmale einzelner oder mehrerer Schutzansprüche untereinander und/oder mit einzelnen oder mehreren Merkmalen der Ausführungsbeispiele.

Es zeigt:
- Fig. 1: einen schematischen Aufbau einer ersten Ausführungsvariante einer erfindungsgemäßen Oligonukleotidsonden-Kombination, mit einer als lineare Primärsonde ausgestalteten Primärsonde und einer als lineare Sekundärsonde ausgestalteten Sekundärsonde, die über komplementäre Basenpaare miteinander verbunden sind, wobei die Primärsonde einen einzigen Köderabschnitt aufweist und die Sekundärsonde mit zwei Markierungen versehen ist,
- Fig. 2: einen weiteren schematischen Aufbau einer zweiten Ausführungsvariante einer erfindungsgemäßen Oligonukleotidsonden-Kombination, mit einer als lineare Primärsonde ausgestalteten Primärsonde und einer als lineare Sekundärsonde ausgestalteten Sekundärsonde, die über komplementäre Basenpaare miteinander verbunden sind, wobei die Primärsonde zwei Köderabschnitte aufweist und die Sekundärsonde mit zwei Markierungen versehen ist,
- Fig. 3: einen weiteren schematischen Aufbau einer dritten Ausführungsvariante einer erfindungsgemäßen Oligonukleotidsonden-Kombination, mit einer als Haarnadel-Primärsonde ausgestalteten Primärsonde mit interner Haarnadelstruktur und einer als lineare Sekundärsonde ausgestalteten Sekundärsonde, die über komplementäre Basenpaare miteinander verbunden sind, wobei die Primärsonde einen einzigen Köderabschnitt aufweist und die Sekundärsonde mit zwei Markierungen versehen ist,
- Fig. 4: einen weiteren schematischen Aufbau einer vierten Ausführungsvariante einer erfindungsgemäßen Oligonukleotidsonden-Kombination, mit einer als Haarnadel-Primärsonde ausgestalteten Primärsonde mit interner Haarnadelstruktur und einer als lineare Sekundärsonde ausgestalteten Sekundärsonde, die über komplementäre Basenpaare miteinander verbunden sind, wobei die Primärsonde einen einzigen Köderabschnitt und einen Quencher aufweist, und wobei die Sekundärsonde mit zwei Markierungen versehen ist,
- Fig. 5: einen schematischen Aufbau einer Primärsonde mit interner Haarnadelstruktur und einem einzigen Köderabschnitt,
- Fig. 6: einen weiteren schematischen Aufbau einer Primärsonde mit interner Haarnadelstruktur und zwei Köderabschnitten,
- Fig. 7: einige Beispiele für Sekundärsonden 6 und
- Fig. 8: einige Beispiele für Primärsonden 5.

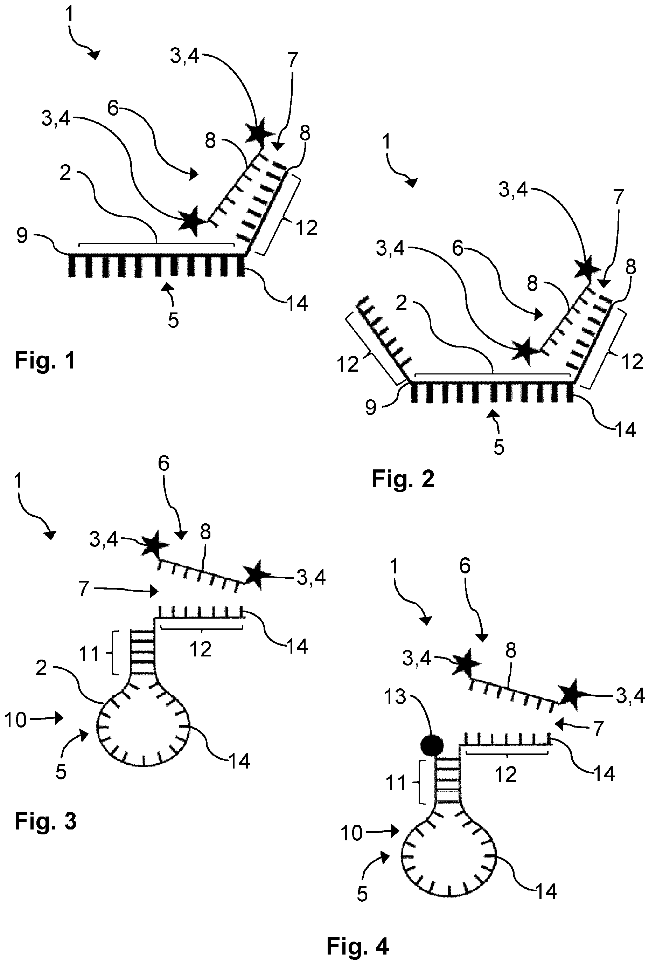

In den Figuren 1 bis 4 sind vier mögliche Ausführungsbeispiele einer erfindungsgemäßen Oligonukleotidsonden-Kombination in vereinfachter schematischer Form dargestellt, wobei die Oligonukleotidsonden-Kombination im Ganzen jeweils als 1 bezeichnet ist.

Die Oligonukleotidsonden-Kombination 1 ist dazu ausgelegt, ein Nachweisverfahren zur Erkennung bestimmter Mikroorganismen vorzunehmen. Die Oligonukleotidsonden-Kombination 1 bindet dabei spezifisch über einen Funktionsabschnitt 2 (Funktionssequenz) an eine vorzugsweise komplementäre Zielsequenz des zu detektierenden Mikroorganismus.

Um einen Nachweis vornehmen zu können, weist die Oligonukleotidsonden-Kombination 1 wenigstens eine detektierbare Markierung 3 auf. Hierbei kann es sich zum Beispiel um eine Farbmarkierung 4 handeln, die einen optischen Nachweis ermöglicht.

Die Oligonukleotidsonden-Kombination 1 weist wenigstens eine Primärsonde 5 und wenigstens eine Sekundärsonde 6 auf, die unterschiedliche Funktionen aufweisen, wie es nachfolgend noch ausführlich erläutert wird. Der Begriff "Sonde" kann sich im Sinne der Erfindung auf ein Oligonukleotid beziehen.

Durch die zuvor genannte Markierung 3 wird vorzugsweise erst dann ein detektierbares Signal erzeugt, wenn es zu einer spezifischen Anbindung des Funktionsabschnitts 2 an die Zielsequenz kam. Durch diese Anbindung kann eine Interaktion zwischen der Primärsonde 5 und der Sekundärsonde 6 ein detektierbares Signal der Markierung 3 auslösen. Bei dem Signal kann es sich zum Beispiel um ein optisch erfassbares Signal, wie eine Farbreaktion, handeln.

Um eine Detektion einer spezifischen Anbindung der Oligonukleotidsonden-Kombination 1 an eine Zielsequenz detektieren zu können, müssen die Primärsonde 5 und die Sekundärsonde 6 miteinander verbunden sein. Die Verbindung kann hierbei zum Beispiel über komplementäre Basenpaare 7 der jeweils als Kette 8 ausgebildeten Teile 5 und 6 hergestellt sein.

Die Primärsonde 5 weist den oben genannten Funktionsabschnitt 2 auf, der an die Zielsequenz des Mikroorganismus bindet. Die Primärsonde 5 kann zum Beispiel dazu ausgelegt sein, um einen Nachweis von rRNA Sequenzen spezifisch für die Bakteriengattung "Enterobacteriacaen" zu ermöglichen.

Die als Oligonukleotid ausgebildete Primärsonde 5 kann beispielsweise folgendermaßen aufgebaut sein. Es enthält einen Nukleinsäure-Sequenzabschnitt (Funktionsabschnitt 2), der revers-komplementär zu einer Nukleinsäure der Zielsequenz ist, die in einem Mikroorganismus nachgewiesen werden soll. Unter den gegebenen Assay Bedingungen binden diese aneinander (d.h. ausreichend hohe Schmelztemperatur). Unter den gegebenen Assay-Bedingungen bindet die Primärsonde 5 nicht in nah verwandten Mikroorganismen, die nicht nachgewiesen werden sollen (vorzugsweise mindestens 2 Mismatches). Es enthält einen zweiten Sequenzabschnitt (Köderabschnitt 12), der revers-komplementär zu einem Nukleinsäuresequenz-Abschnitt der Sekundärsonde 6 ist. Unter den gegebenen Assay-Bedingungen binden diese aneinander (d.h. ausreichend hohe Schmelztemperatur). Dieser Sequenzabschnitt (Köderabschnitt 12) bindet nicht in der Zielsequenz. Dieser Köderabschnitt kann am 3' und/oder am 5' Ende der Primärsonde 5 vorliegen. Der "Köderabschnitt" kann durch einen "Linker" aus einem oder mehreren Nukleotiden vom Rest der Sonde (erster Sequenzabschnitt), insbesondere vom Funktionsabschnitt 2, distanziert sein. Der zweite Sequenzabschnitt (Köderabschnitt 12) kann derart ausgebildet sein, dass er keine Komplementarität innerhalb der Primärsonde länger als fünf Basen in einer Abfolge aufweist.

Die als Oligonukleotid ausgebildete Sekundärsonde 6 weist die oben genannte wenigstens eine Markierung 3 auf, durch welche ein detektierbares Signal erzeugbar ist. Die Sekundärsonde 6 weist die Eigenschaften auf, dass sie einen Nukleinsäure-Sequenzabschnitt enthält, der revers-komplementär zu dem dazu passenden Nukleinsäuresequenz-Abschnitt (Köderabschnitt 12) der Primärsonde 5 ist. Die Schmelztemperatur der Sekundärsonde 6 ist so gewählt, dass unter den gegebenen Assay-Bedingungen diese mit dem komplementären Abschnitt der Primärsonde 5 hybridisiert. Diese Schmelztemperatur liegt dabei bei gleich oder höher als die für das Hybrid der Primärsonde mit der Zielsequenz des Mikroorganismus. Die Sekundärsonde 6 kann maximal 25 Basen aufweisen. Die Sekundärsonde 6 bindet unter den gegebenen Assay-Bindungen nicht in einer der Zielsequenzen, an andere Stellen im Mikroorganismus und/oder an deren reverse-Komplement (vorzugsweise liegen mindestens 2 Mismatches vor). Die Sekundärsonde 6 setzt sich aus einer Abfolge von maximal drei der vier möglichen Basen (G,T,C,A) zusammen. Die Basen G und C werden nicht in derselben Sequenz verwendet (d.h. entweder/oder). Der ausschließliche Aufbau der Sekundärsonde 6 kann aus den Basen A und G (Heteropurin-Sequenz) mit mehreren abwechselnden Repeats von 2 bis 5 gleichen Basen (z.B. AAGGAAGGAA) sein, da dies zu einer stark gesenkten Affinität gegenüber RNA-Sequenzen führt und so das "Off-Target"-Risiko (bzw. Falsch-Positive) gesenkt wird. Die Sequenz der Sekundärsonde 6 kann so ausgebildet sein, dass keine Selbstkomplementarität von mehr als 4 Basen in einer Abfolge vorliegt. Ein bis mehrere Nukleotide der Sekundärsonde 6 können zum Zweck einer höheren Bindungsaffinität und/oder einer höheren Schmelztemperatur und/oder einer Verkürzung der Sondenlänge als modifizierte Nukleotide ausgebildet sein. Die Modifikationen können die Zuckerreste der Nucleotide, die Basen oder die Phosphat-Reste betreffen. Vorzugsweise sind die modifizierten Nukleotide sogenannte "Locked nucleid acids" (LNA)-Nukleotide. Die LNA-Bausteine sollten dabei nicht selbst-komplementär sein.

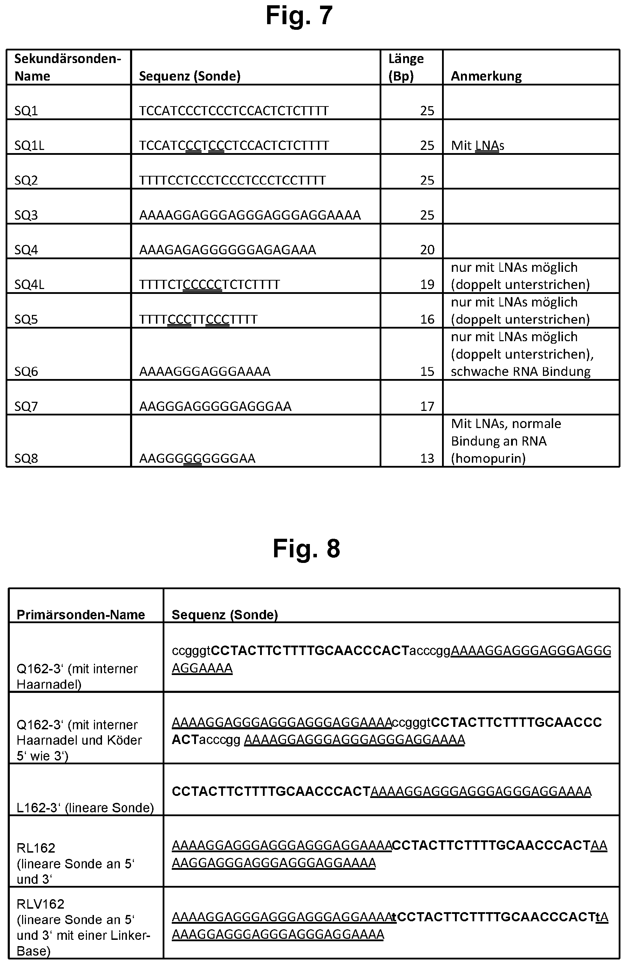

In der Figur 7 sind Beispiele für Sekundärsonde 6 dargestellt, wobei die LNAs doppelt unterstrichen sind. Figur 8 zeigt einige Beispiele für Primärsonde 5. Die gesamte Sequenz besteht aus Reverse-komplementärem Abschnitt der Zielsequenz (Großbuchstaben), der Ködersequenz (Großbuchstaben unterstrichen) und der Stammsequenzen (Kleinbuchstaben) oder einem Linker-Nukleotid (Kleinbuchstaben unterstrichen).

Die Verwendung einer Sekundärsonde 6 ermöglicht die Reduktion der Zahl der nötigen Produktionsentwicklungsprozesse für Marker-modifizierte Oligonukleotide auf einen einzigen Herstellungsprozess. Es ermöglicht zudem ein vereinfachtes Screening nach geeigneten Zielsequenzen. So ist zum Beispiel der gleichzeitige Nachweis mehrerer Zielsequenzen vereinfacht, was beispielsweise die Detektierbarkeit von Bakterien, wie Enterobacteriaceaen, erheblich erhöht, da eine höhere Signalintensität, wie Fluoreszenzintensität, pro Bakterium (d.h. besseres Signal-Rausch Verhältnis) erreicht werden kann. Die wenigstens eine Markierung 3 kann einen Marker oder eine Kombination aus wenigstens zwei Markern aus der Gruppe von Fluoreszenz-basierter, Biotin- oder Radioaktivitätsmarkierung, einem Affinitätsmarker und/oder einer enzymatisch aktiven Gruppe aufweisen. Die Primärsonde 5 kann alternativ oder ergänzend dazu mit einem Farbstoff oder Quencher 13 am 5' oder 3' Endekonjugiert sein, wie in Fig. 4 gezeigt ist.

In den Figuren 1 und 2 ist eine Primärsonde 5 gezeigt, das als lineare Sonde 9 ausgebildet ist, da die einzelnen Nukleinbasen 14 der Primärsonde 5 nicht miteinander verbunden sind, also nicht an andere Nukleinbasen 14 der Primärsonde 5 angebunden sind, und aufgrund ihrer Sequenzabfolge keine internen Sekundärstrukturen ausbilden können.

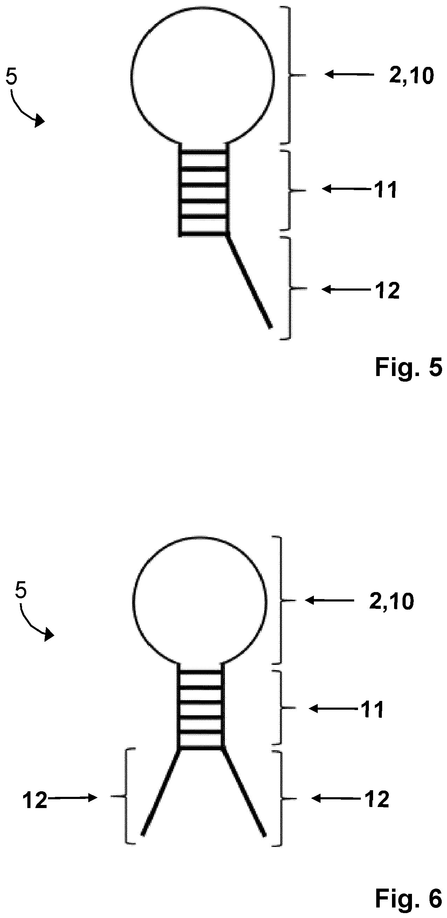

In den Figuren 3-6 ist eine weitere Ausführungsvariante einer Primärsonde 5 gezeigt, die als Haarnadelsonde 10 (Haarnadelprimärsonde) ausgebildet ist. Die Haarnadelsonde 10 weist einen Stammabschnitt 11 auf, in welchem Nukleinbasen 14 der Primärsonde 5 aneinander angebunden sind, so dass der Funktionsabschnitt 2 den Loop einer Haarnadelstruktur 10 ausbildet.

Die als Haarnadelsonde ausgebildete Primärsonde 5 enthält zwei zueinander komplementäre Nukleinsäuren-Sequenzsabschnitte (insbesondere 5 bis 8 Basen lang), die unter den gegebenen Assay-Bedingungen aneinanderbinden und so eine Haarnadel-Struktur ermöglichen. Diese beiden Sequenzabschnitte bilden hierbei den zuvor bereits erwähnten Stammabschnitt 11 (Stem" bzw. "Stamm") der Haarnadel-Struktur. Es kann sich so eine interne Haarnadel-Struktur bilden. Die Sequenz des Köderabschnitts 12 kann dabei so gewählt sein, dass sie außerhalb dieser Haarnadel-Struktur liegt und nicht daran beteiligt ist (siehe Fig. 3-6). Die Haarnadel-Struktur ermöglicht sowohl eine bessere Mismatch-Unterscheidung als auch den zusätzlichen Einbau eines Quenchers 13 in die Primärsonde.

Die hierin beschriebene und/oder beanspruchte Oligonukleotidsonden-Kombination 1 eignet sich somit insbesondere zur Verwendung in einem Verfahren zum spezifischen Nachweis von Mikroorganismen in einer Probe mittels Fluoreszenz-In-Situ-Hybridisierung (FISH), wie es hierin beschrieben und/oder beansprucht ist.

Die Erfindung betrifft also insbesondere eine Oligonukleotidsonden-Kombination 1 zum spezifischen Nachweis von Mikroorganismen, mit einer Primärsonde 5 und einer Sekundärsonde 6, wobei die Primärsonde 5 wenigstens einen Funktionsabschnitt 2 und die Sekundärsonde 6 wenigstens eine ein detektierbares Signal erzeugende Markierung 3 aufweist, wobei der Funktionsabschnitt 2 an eine Zielsequenz eines nachzuweisenden Mikroorganismus spezifisch anbindet, und wobei die Primärsonde 5 und die Sekundärsonde 6 miteinander verbunden sind aber getrennte Ketten 8 darstellen.

### Bezugszeichenliste

1 Oligonukleotidsonden-Kombination
2 Funktionsabschnitt; Funktionssequenz
3 Markierung
4 Farbmarkierung
5 Primärsonde
6 Sekundärsonde
7 komplementäre Basenpaare
8 Kette
9 lineare Sonde
10 Haarnadel-Sonde; Haarnadel-Struktur
11 Stammabschnitt; Stammsequenz
12 Köderabschnitt; Ködersequenz
13 Quencher
14 Nukleinbase

## Patentansprüche

1. Oligonukleotidsonden-Kombination (1) zum spezifischen Nachweis von Mikroorganismen, insbesondere zur Detektion von wenigstens einer Zielsequenz, mit wenigstens einem Funktionsabschnitt (2) und wenigstens einer detektierbaren Markierung (3), insbesondere einer Farbmarkierung (4), wobei der wenigstens eine Funktionsabschnitt (2) an die Zielsequenz von Mikroorganismen der nachzuweisenden Gruppe spezifisch anbindet und durch eine Interaktion mit der wenigstens einen Markierung (3) ein detektierbares Signal auslöst, **dadurch gekennzeichnet, dass** die Oligonukleotidsonden-Kombination (1) wenigstens eine Primärsonde (5) und wenigstens eine Sekundärsonde (6) aufweist, die, vorzugsweise über komplementäre Basenpaare (7), miteinander verbunden sind, aber getrennte Ketten (8) darstellen, wobei an der wenigstens einen Primärsonde (5) der wenigstens eine Funktionsabschnitt (2) und an der wenigstens einen Sekundärsonde (6) die wenigstens eine Markierung (3) ausgebildet sind.

2. Oligonukleotidsonden-Kombination (1) nach Anspruch 1, **dadurch gekennzeichnet, dass** die wenigstens eine Primärsonde (5) als lineare Sonde (9) und/oder Haarnadel-Sonde (10) ausgebildet ist, insbesondere wobei die wenigstens als eine Haarnadel-Sonde (10) ausgebildete Primärsonde (5) einen Stammabschnitt (11) aufweist, der zur Bildung der Haarnadel-Struktur (10) ausgebildet ist.

3. Oligonukleotidsonden-Kombination (1) nach einem der vorangehenden Ansprüche, **dadurch gekennzeichnet, dass** die wenigstens eine Primärsonde (5) wenigstens einen Köderabschnitt (12) aufweist, der mit dem Stammabschnitt (11) übereinstimmend oder separat, insbesondere beabstandet, von dem Stammabschnitt (11) ausgebildet ist.

4. Oligonukleotidsonden-Kombination (1) nach einem der vorangehenden Ansprüche, **dadurch gekennzeichnet, dass** ein oder der Köderabschnitt (12) an wenigstens einem Ende der wenigstens einen Primärsonde (5) ausgebildet ist.

5. Oligonukleotidsonden-Kombination (1) nach einem der vorangehenden Ansprüche, **dadurch gekennzeichnet, dass** die wenigstens eine Primärsonde (5) einen Quencher (13) aufweist, der zur Deaktivierung der wenigstens einen Markierung (3) der wenigstens einen Sekundärsonde (6) ausgebildet ist.

6. Oligonukleotidsonden-Kombination (1) nach einem der vorangehenden Ansprüche, **dadurch gekennzeichnet, dass** die wenigstens eine Primärsonde (5) einen oder den Köderabschnitt (12) aufweist, der an die wenigstens eine Sekundärsonde (6) anbindet.

7. Oligonukleotidsonden-Kombination (1) nach einem der vorangehenden Ansprüche, **dadurch gekennzeichnet, dass** die wenigstens eine Sekundärsonde (6) aus DNA-Nukleotiden oder aus einer Mischung von DNA-Nukleotiden und wenigstens einem modifizierten Nukleotid, vorzugsweise einem LNA-Nukleotid, besteht, insbesondere wobei das wenigstens eine modifizierte Nukleotid der wenigstens einen Sekundärsonde (6) und wenigstens ein modifiziertes Nukleotid der wenigstens einen Primärsonde (5) untereinander nicht komplementär ausgebildet sind.

8. Oligonukleotidsonden-Kombination (1) nach einem der vorangehenden Ansprüche, **dadurch gekennzeichnet, dass** die wenigstens eine Sekundärsonde (6) eine Länge von weniger als 25 Nukleinbasen (14) aufweist.

9. Oligonukleotidsonden-Kombination (1) nach einem der vorangehenden Ansprüche, **dadurch gekennzeichnet, dass** die wenigstens eine Markierung (3) einen Marker oder eine Kombination aus wenigstens zwei Markern aus der Gruppe von Fluoreszenz-basierter, Biotin- oder Radioaktivitätsmarkierung, einem Affinitätsmarker und/oder einer enzymatisch aktiven Gruppe umfasst.

10. Oligonukleotidsonden-Kombination (1) nach einem der vorangehenden Ansprüche, **dadurch gekennzeichnet, dass** die wenigstens eine Zielsequenz eine oder mehrere verschiedene DNA- und/oder RNA-Sequenz/Sequenzen umfasst.

11. Verfahren zum spezifischen Nachweis von Mikroorganismen in einer Probe mittels In-situ Hybridisierung (ISH), insbesondere Fluoreszenz-In-Situ-Hybridisierung (FISH), umfassend die folgenden Schritte: a) Permeabilisierung und Fixierung der in der Probe enthaltenen Mikroorganismen; b) Inkubieren der fixierten Mikroorganismen mit wenigstens einer Oligonukleotidsonden-Kombination (1) nach einem der Ansprüche 1 bis 10, wobei die einzelne Oligonukleotidsonden-Kombination (1) mit mehreren verschiedenen Nukleinsäuren der Mikroorganismen eine komplementäre Hybridisierung eingeht; c) vorzugsweise optisches Detektieren, durch Fluoreszenzmessung oder Luciferaseaktivität mit vorzugsweise Renilla-luceferin-2-monooxygenase, der in den einzelnen Mikroorganismen erzeugten Hybridisierungen.

12. Verfahren nach Anspruch 11, **dadurch gekennzeichnet, dass** das Detektieren einen Schritt des Quantifizierens mit hybridisierten Oligonukleotidsonden-Kombinationen (1) umfasst.

13. Verfahren nach einem der Ansprüche 11 bis 12, **dadurch gekennzeichnet, dass** jede Oligonukleotidsonden-Kombination (1) nachweisbar nur an die komplementären Zielsequenzen bindet.

14. Verwendung wenigstens einer Oligonukleotidsonden-Kombination (1) nach einem der Ansprüche 1 bis 10 zum spezifischen Nachweis von mehreren verschiedenen Nukleinsäuresequenzen, wobei der Nachweis in einer natürlichen Probe ausgewählt aus klinischen Proben, Lebensmittelproben, Umweltproben, Umgebungsproben und/oder veterinärdiagnostischen Proben und/oder in einer Laborkultur erfolgt.

15. Verwendung eines Probenträgers, insbesondere mit einer Mikrofluidik, zur Ausführung eines Verfahrens nach einem der Ansprüche 11 bis 13 und/oder bei einer Verwendung nach Anspruch 14, insbesondere wobei die Permeabilisierung und/oder die Fixierung und/oder die Hybridisierung und/oder das Detektieren der Markierung im Probenträger erfolgt.


SEQUENCE LISTING

<110> Testo SE & Co. KGaA

<120> Oligonukleotidsonde zum spezifischen Nachweis von Mikroorganismen,
korrespondierendes Verfahren und Verwendung

<130> E 21 0173 C

<160> 15

<170> BiSSAP 1.3.6

<210> 1
<211> 25
<212> RNA
<213> Artificial Sequence

<220>
<223> Sekundärsonde SQ1

<400> 1
tccatccctc cctccactct ctttt                                          25

<210> 2
<211> 25
<212> RNA
<213> Artificial Sequence

<220>
<223> Sekundärsonde SQ1L

<400> 2
tccatccctc cctccactct ctttt                                          25

<210> 3
<211> 25
<212> RNA
<213> Artificial Sequence

<220>
<223> Sekundärsonde SQ2

<400> 3
ttttcctccc tccctccctc ctttt                                          25

<210> 4
<211> 25
<212> RNA
<213> Artificial Sequence

<220>
<223> Sekundärsonde SQ3

<400> 4
aaaaggaggg agggagggag gaaaa                                          25

<210> 5
<211> 20
<212> RNA
<213> Artificial Sequence

<220>
<223> Sekundärsonde SQ4

<400> 5
aaagagaggg gggagagaaa 20

<210> 6
<211> 19
<212> RNA
<213> Artificial Sequence

<220>
<223> Sekundärsonde SQ4L

<400> 6
ttttctcccc ctctctttt 19

<210> 7
<211> 16
<212> RNA
<213> Artificial Sequence

<220>
<223> Sekundärsonde SQ5

<400> 7
ttttcccttc cctttt 16

<210> 8
<211> 15
<212> RNA
<213> Artificial Sequence

<220>
<223> Sekundärsonde SQ6

<400> 8
aaaagggagg gaaaa 15

<210> 9
<211> 17
<212> RNA
<213> Artificial Sequence

<220>
<223> Sekundärsonde SQ7

<400> 9
aagggagggg gagggaa                                                    17

<210> 10
<211> 13
<212> RNA
<213> Artificial Sequence

<220>
<223> Sekundärsonde SQ8

<400> 10
aagggggggg gaa                                                        13

<210> 11
<211> 59
<212> RNA
<213> Artificial Sequence

<220>
<223> Primärsonde Q162-3′ (mit interner Haarnadelstruktur)

<400> 11
ccgggtccta cttcttttgc aacccactac ccggaaaagg agggagggag ggaggaaaa      59

<210> 12
<211> 84
<212> RNA
<213> Artificial Sequence

<220>
<223> Primärsonde Q162-3′ (mit interner Haanadel und Köder 5′ wie 3′)

<400> 12
aaaaggaggg agggagggag gaaaaccggg tcctacttct tttgcaaccc actacccgga      60

aaaggaggga gggagggagg aaaa                                             84

<210> 13
<211> 47
<212> RNA
<213> Artificial Sequence

<220>
<223> Primärsonde L162-3′ (lineare Sonde)

<400> 13
cctacttctt ttgcaaccca ctaaaaggag ggagggaggg aggaaaa                    47

<210> 14
<211> 72
<212> RNA
<213> Artificial Sequence

<220>
<223> Primärsonde RL162 (lineare Sonde an 5′ und 3′)

<400> 14
aaaaggaggg agggagggag gaaaacctac ttcttttgca acccactaaa aggagggagg     60

gagggaggaa aa                                                         72



<210> 15
<211> 74
<212> RNA
<213> Artificial Sequence



<220>
<223> Primärsonde RLV162 (lineare Sonde an 5′ und 3′ mit einer
      Linker-Base)

<400> 15
aaaaggaggg agggagggag gaaaatccta cttcttttgc aacccactta aaaggaggga     60

gggagggagg aaaa                                                       74